(19) Europäisches Patentamt
European Patent Office
Office européen des brevets

(11) Publication number: 0 439 212 A1

# (12) EUROPEAN PATENT APPLICATION

(21) Application number: 91200047.8

(22) Date of filing: 12.01.91

(51) Int. Cl.5: G01N 33/569, G01N 33/543, G01N 33/546

(30) Priority: 22.01.90 US 468046
22.01.90 US 468047
22.01.90 US 468395

(43) Date of publication of application:
31.07.91 Bulletin 91/31

(84) Designated Contracting States:
CH DE FR GB IT LI NL SE

(71) Applicant: EASTMAN KODAK COMPANY
343 State Street
Rochester, NY 14650-0221(US)

(72) Inventor: Snyder, Brian Anthony, c/o
EASTMAN KODAK COMPANY
Patent Department 343 State Street
Rochester New York 14650-2201(US)
Inventor: Abrams, Catherine Theresa, c/o
EASTMAN KODAK COMP.
Patent Department 343 State Street
Rochester New York 14650-2201(US)
Inventor: Zambon, Joseph James, c/o
EASTMAN KODAK COMP.
Patent Department 343 State Street
Rochester New York 14650-2201(US)
Inventor: Fisher, John Gary, c/o EASTMAN
KODAK COMP.
Patent Department 343 State Street
Rochester New York 14650-2201(US)
Inventor: Grogan, Elizabeth Ann, c/o
EASTMAN KODAK COMP.
Patent Department 343 State Street
Rochester New York 14650-2201(US)
Inventor: Reynolds, Homer Stanley, c/o
EASTMAN KODAK COMP.
Patent Department 343 State Street
Rochester New York 14650-2201(US)
Inventor: Contestable, Paul Bernard, c/o
EASTMAN KODAK COMP.
Patent Department 343 State Street
Rochester New York 14650-2201(US)

(74) Representative: Nunney, Ronald Frederick
Adolphe et al
Kodak Limited Patent Department Headstone
Drive
Harrow Middlesex HA1 4TY(GB)

## (54) Article, test kit and sandwich assay for the detection of bacteroides intermedius, bacteroides gingivalis or actinobacillus actinomycetemcomitans.

(57) One or more serotypes of any of the microorganisms Bacteroides intermedius, Bacteroides gingivalis and Actinobacillus actinomycetemcomitans can be sensitively and selectively detected in a sandwich assay. This method comprises contacting a specimen containing antigen extracted from the microorganism with a microporous membrane having reactive antibodies affixed thereto as a water-insoluble reagent, and contacting the same antigen with detectably labeled antibodies thereby forming a immunological sandwich complex bound to the membrane. After unbound materials are washed away, the bound complex can be appropriately detected. The water-insoluble reagent is composed of particles to which the antibodies are bound. The particles are substantially on the surface of the membrane as opposed to being embedded therein. This assay enables detection of the microorganism without significant cross-reactivity with related organisms.

EP 0 439 212 A1

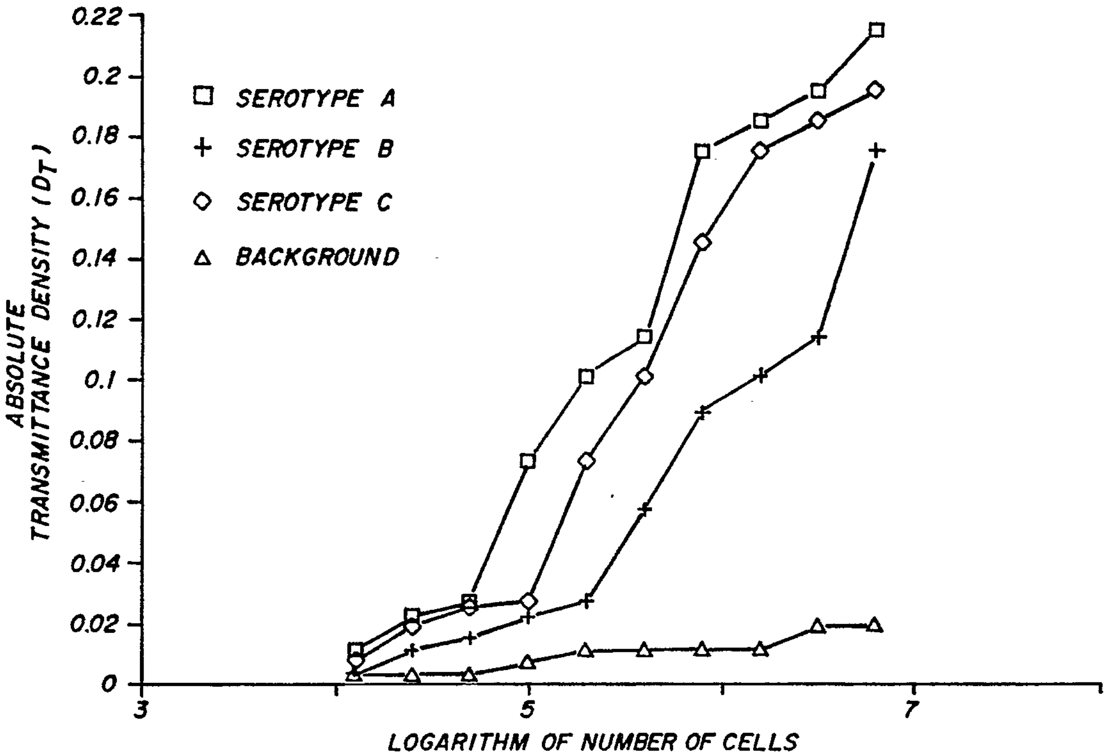
0.22
0.2
0.18
0.16
0.14
0.12
0.1
0.08
0.06
0.04
0.02
0
ABSOLUTE
TRANSMITTANCE DENSITY ($D_T$)
□ SEROTYPE A
+ SEROTYPE B
◇ SEROTYPE C
△ BACKGROUND
3
5
7
LOGARITHM OF NUMBER OF CELLS

FIG. 1

# ARTICLE, TEST KIT AND SANDWICH ASSAY FOR THE DETECTION OF BACTEROIDES INTERMEDIUS, BACTEROIDES GINGIVALIS OR ACTINOBACILLUS ACTINOMYCETEMCOMITANS

This invention relates to a sandwich assay useful for the detection of any or all of the three serotypes (A, B and C) of any of the microorganisms Bacteroides intermedius, Bacteroides gingivalis and Actinobacillus actinomycetemcomitans. An article and test kit useful in the assay are also provided. This invention is useful in dental research and health care.

There is a continuing need in medical, dental and veterinary practices, in research and diagnostic procedures, and for rapid and accurate detection or quantification of biological or chemical substances present in biological fluids or specimens. For example, the presence of various microorganisms in human and animal tissues, fluids or cells is very important for diagnosis and effective treatment of diseases.

Specific microorganisms have been implicated as markers for a number of periodontal diseases in humans and animals, such as gingivitis and periodontitis. The importance of such diseases is growing in the human population, especially as people live longer, and prevention thereof is becoming of considerable medical and commercial importance. In addition, the proper care of animals (including dental care) is a growing concern in our culture.

Detection of microorganisms associated with periodontal diseases has been accomplished using culture techniques (which are generally slow), DNA probes and a number of immunological procedures, such as agglutination assays, enzyme linked immunosorbent assays (ELISA), immunofluorescence and immunoprecipitation. The immunological procedures use immunological reactions of an antigenic site of the microorganism (which may be on a component extracted therefrom) with a corresponding antibody specific thereto. The resulting immunological reaction complex can then be detected in a number of ways.

The black-pigmented Bacteroides species are gram negative, anaerobic rods which are common in the etiology of various periodontal diseases, odontogenic abscesses and endodontic lesions. Such species include B. intermedius, B. gingivalis, B. forsythus and B. endodontalis. B. gingivalis is sometimes identified in the art as Bacteroides-(Porphyromonas) gingivalis or as Porphyromonas gingivalis. As defined herein, periodontal disease refers to a wide variety of diseases in humans and animals which occur in the periodontal area of the oral cavity, and includes diseases affecting the connective tissue, loss of alveolar bone as well as the gingiva. It includes diseases that may occur at different times of human life, such as in infancy, youth, pregnancy or old age.

It is also known that certain serogroups or serotypes within a bacterial species are associated with certain periodontal diseases. For example, it has been found [Nakazawa et al, Infect. Immun., 56(6), pp. 1647-1651, 1988] that serotype B of B. intermedius is primarily found in human adolescent periodontitis, while serotypes B and C are responsible for adult periodontitis.

Actinobacillus actinomycetemcomitans - (identified herein as A. actinomycetemcomitans) is an oral gram-negative facultative organism which is closely related to the Haemophilus group of organisms. This organism can cause severe human infections, including bacterial endocarditis, thyroid gland abscess, urinary tract infection, brain abscess and vertebral osteomyelitis. It has been strongly implicated in the pathogenesis of certain types of periodontal disease, particularly in localized juvenile periodontitis and also in some forms of adult periodontitis. Human isolates of A. actinomycetemcomitans have been divided into three major serogroups designated as serotypes A, B and C, corresponding to strains designated as 75 (ATCC 43717), Y4 (ATCC 43718) and 67 (ATCC 43719), respectively, by Zambon et al (Infect. Immun., 41, pp. 19-27, 1983).

Clinical assays specific to such bacteria in gingival crevicular fluid and subgingival dental plaque are useful in the diagnosis of periodontal disease, in evaluating the options for and progress of periodontal treatment, and in determining the status of the patient at later dental examinations. The standard culture techniques used to identify such organisms are time consuming, expensive and require a high level of operator expertise. Such tests also may lack sufficient sensitivity for detection of low levels of organisms due to strict anaerobic conditions required during transport.

The various immunoassays noted above have been developed and used with some success. Particularly useful are radioimmunoprecipitation assays and ELISA tests. US-A-4,741,999 describes monoclonal antibodies to antigens of A. actinomycetemcomitans and their use in ELISA assays. Monoclonal and polyclonal antibodies to various Bacteroides species have been prepared for similar assays [see for example, Nakazawa et al, noted above and Zambon et al, J.Periodon., 56-(Supp), pp. 32-40, 1985].

Generally, the assays of the art have been slow, tedious and directed to a single organism. In many cases, the assays are highly cross-reactive with related species, and thus have limited usefulness.

A screening assay has been developed for detecting the presence of any of the three microorganisms A. actinomycetemcomitans, B. gingivalis and B. intermedius. This screening method is highly useful for practitioners so they can initially determine if a patient needs periodontal treatment, but it does not indicate which microorganism may be present.

Once screening has been done, and the results are found to be positive, it would be desirable to be able to determine which microorganisms are present so treatment can be prescribed specifically. Such treatment could then begin early. Presently, any differentiation would have to be carried out using tedious culture techniques, DNA probe technology or the undesirable immunological methods described above.

One method of determining the presence of a microorganism is known as a "direct binding" assay whereby the antigen of interest is directly bound to a solid substrate for later complexation with the appropriate detectable antibodies. While this assay is advantageous over the known assays in speed and sensitivity, there is still improvement desired in the area of specificity. For example, it was found that the cross-reactivity with antigenic sites on Haemophilus organisms was unacceptably high using a direct binding assay. This cross-reactivity is not acceptable for providing a highly sensitive and reliable test for practitioners in dental health care. Moreover, the direct binding assay is not as sensitive to low levels of A. actinomycetemcomitans as is desired for very early detection and preventive dental care. Thus, it would be desirable to have an assay exhibiting reduced cross-reactivity with related species when a practitioner wants to assay a patient sample for individual microorganisms associated with periodontal diseases.

The problems described above are solved with a method for the determination of one or more serotypes of any of the microorganisms Bacteroides intermedius, Bacteroides gingivalis and Actinobacillus actinomycetemcomitans comprising the steps of:

A. contacting an aqueous specimen suspected of containing antigen extracted from one or more serotypes of any of the microorganisms Bacteroides intermedius, Bacteroides gingivalis and Actinobacillus actinomycetemcomitans with a microporous membrane having thereon a water-insoluble reagent comprising water-insoluble particles having affixed thereto antibodies to the antigen, the reagent being substantially all on the surface of the membrane,

to form a water-insoluble immunological complex between the antibodies and extracted antigen present in the specimen on the membrane,

B. prior to, simultaneously with or subsequent to the contact in step A, contacting the extracted antigen with a detectably labeled, water-soluble antibody to the antigen so as to form a labeled water-insoluble immunological complex of both the labeled and water-insoluble antibodies with extracted antigen in the specimen on the membrane,

C. simultaneously with or subsequently to the contacting in step B, separating the labeled water-insoluble complex from uncomplexed materials by washing the uncomplexed materials through the microporous membrane, and

D. detecting the labeled complex on the membrane as an indication of the presence of the antigen in the specimen.

Moreover, a water-insoluble article of this invention comprises a microporous membrane having affixed to at least one of its surfaces a water-insoluble reagent comprising water-insoluble particles,

the article characterized wherein the particles have affixed thereto antibodies directed to at least one serotype of any of the microorganisms Bacteroides intermedius, Bacteroides gingivalis and Actinobacillus actinomycetemcomitans, and the reagent is substantially all on the membrane surface.

Further, a test kit for the detection of one or more serotypes of any of the microorganisms Bacteroides intermedius, Bacteroides gingivalis and Actinobacillus actinomycetemcomitans comprises:

a. a water-insoluble article comprising a microporous membrane, having affixed to at least one of its surfaces, a water-insoluble reagent comprising water-insoluble particles, and

b. detectably labeled antibodies, the kit characterized wherein the particles have affixed thereto antibodies directed to at least one serotype of any of the microorganisms Bacteroides intermedius, Bacteroides gingivalis and Actinobacillus actinomycetemcomitans, the detectably labeled antibodies are directed to the same serotype of Bacteroides intermedius, Bacteroides gingivalis of Actinobacillus actinomycetemcomitans as the antibodies affixed to the water-insoluble particles, and the reagent is substantially all on the membrane surface.

The present invention provides a rapid and sensitive method for detecting any or all serotypes of any of the microorganisms B. intermedius, B. gingivalis or A. actinomycetemcomitans in periodontal biological specimens. The method can provide a definitive result within a few minutes so the that practitioner can make a quick assessment of the patient's need for periodontal treatment. In addition, each of the three serotypes A, B and C of each microorganism can generally be differentiated in a single test device with the present invention even though some cross-reactivity between serotype A and serotype C antibodies may be seen.

Most importantly, the assay of this invention for either B. intermedius or B. gingivalis shows minimal cross-reactivity with the similar Bacteroides organisms (such as B. oralis, B. asaccharoliticus or B. loescheii). Thus, selectivity of the assay is significantly better than that of other assays (such as the "direct binding" assay). This improvement in selectivity is achieved without having to remove cross-reactive antibodies from the test specimen, as is the normal case with prior art methods. Thus, maximum sensitivity is maintained without sacrificing assay specificity.

Further, the present invention provides a rapid and sensitive method for detecting any or all serotypes of the microorganism A. actinomycetemcomitans in periodontal biological specimens. The assay of this invention shows minimal cross-reactivity with the similar Haemophilus organisms, so that selectivity of the assay is greatly improved over direct binding assays. Equally important, the method of this invention is more sensitive than direct binding assays so that lower cell concentrations can be detected, thereby making earlier preventive dental health care possible.

These advantages are achieved with a sandwich assay method to detect the extracted antigen with the desired selectivity improvements without sacrificing assay sensitivity.

Moreover, the assay is carried out using a microporous membrane to which an insolubilized antibody to the extracted antigen is affixed for complexation. This membrane is also used to separate the resulting insoluble immunological complex from unwanted debris and uncomplexed materials during the assay. For serotype differentiation, the membrane can have distinct regions with the appropriate corresponding antibodies affixed thereto so that the presence or absence of separate signals can be used to differentiate the serotypes.

The water-insoluble reagent used in this invention is not embedded within the pores of the membrane as is taught in the art (for example, in EP-A-0 200 381 and WO-A-87/03690). Assays carried out using embedded reagents are generally less sensitive because the resulting complex is partially hidden from detection reagents by the porous matrix. Improved sensitivity and detectability are needed for highly successful commercial products, and putting the reagents substantially on the surface of a microporous membrane, as in the present invention, provides these desired results.

FIG. 1 is a graphical plot of absolute transmittance density ($D_T$) vs. the logarithm of the number or cells for the simultaneous detection of all B. intermedius serotypes in a sandwich assay. It is described in more detail in Example 1 below.

FIG. 2 is a graphical plot of absolute transmittance density ($D_T$) vs. the logarithm of the number of cells in a sandwich assay to demonstrate the cross-reactivity of antibodies directed to B. intermedius to B. loescheii antigen. It is discussed in more detail in Example 2 below.

FIG. 3 is a graphical plot of absolute transmittance density ($D_T$) vs. the logarithm of the number of cells in a direct binding assay showing the cross-reactivity of antibodies directed to B. intermedius to B. loescheii antigen. It demonstrates the Control assay shown in Example 2 below.

FIG. 4 is a graphical plot of absolute transmittance density ($D_T$) vs. the logarithm of the number or cells for the use of serotype B antibodies to B. intermedius to detect B. intermedius serotypes A, B and C antigens in a sandwich assay. It is described in more detail in Example 3 below.

FIG. 5 is a graphical plot of absolute transmittance density ($D_T$) vs. the logarithm of the number or cells for the use of serotype C antibodies to B. intermedius to detect B. intermedius serotype A and C antigens in a sandwich assay. It is described in more detail in Example 3 below.

FIG. 6 is a graphical plot of absolute transmittance density ($D_T$) vs. the logarithm of absolute cell concentration illustrating data obtained in a sandwich assay of this invention for the detection of A. actinomycetemcomitans. This plot is referred to in Example 4 below.

FIG. 7 is a graphical plot of absolute transmittance density ($D_T$) vs. the logarithm of absolute cell concentration illustrating data obtained in a direct binding assay for A. actinomycetemcomitans which is identified as a comparative control in Example 4 below.

FIGS. 8 and 9 are graphical plots of absolute transmittance density ($D_T$) vs. the logarithm of the number of cells illustrating data obtained in sandwich assays of this invention for A. actinomycetemcomitans. These plots are referred to in Example 5 below.

FIG. 10 is a graphical plot of absolute transmittance density ($D_T$) vs. the logarithm of absolute cell concentration for the detection of all three serotypes of B. gingivalis in a sandwich assay. These data are referred to in Example 6 below.

The present invention can be used to rapidly and sensitively detect antigens (for example lipopolysaccharides, capsule antigens or membrane proteins) of one or more of the three serotypes (A,B and C) of any one of B. intermedius, B. gingivalis or A. actinomycetemcomitans. While such antigens may be detectable as part of intact cells, normally, they are extracted in a suitable manner from the whole cells present in a biological specimen. Such specimens include, but are not limited to, saliva or mucous from the throat or mouth, urine, lacrimal fluid, human or animal tissue extracts, dental plaque and gingival crevicular fluid. Generally, the organism is detected in dental plaque, saliva or gingival crevicular fluid.

Antigen extraction is suitably accomplished using physical or chemical means, such as by use of a detergent (such as sodium dodecyl sulfate, sodium decyl sulfate or sodium deoxycholate) using standard procedures, as described, for example, in US-A-4,741,999, osmotic shock [see for example, Dirienzo et al, Infect. & Immun., 47(1), pp. 31-36, 1985], or sonic means [see for example, Zambon et al, Infect. & Immun., 41(1), pp. 19-27, 1983].

If desired, the antigenic material can be removed from the original specimen, or the original specimen can be suitably diluted with water or a suitable buffer or filtered in order to remove extraneous matter and facilitate complexation of antigen and antibody in the assay.

Antibodies used in the practice of this invention can be monoclonal or polyclonal. Monoclonal and polyclonal antibodies can be prepared using standard procedures, such as those described in US-A-4,741,999. Polyclonal antibodies can also be produced using standard procedures, such as described, for example in the literature articles by Zambon et al and Nakazawa et al (both noted above). Generally, a mammal (such as a rabbit) is immunized one or more times with a suitable quantity of an antigenic component or whole bacterial cells of the organism. After a suitable time, when the titer is acceptable, antisera is recovered from the mammal. Antibodies can be removed from the antisera and purified if desired using known procedures and stored in frozen buffered solutions until used. Further details of such procedures are well known in the art.

Generally, once the antibodies are isolated, they can be stored and used in buffered solutions containing one or more suitable buffers and a preservative if desired. Preferably, they are stored in frozen form. In the buffered solutions, the pH is generally maintained at from 6 to 9, and preferably at from 6.5 to 8.5. Useful buffers include phosphate buffered saline solution, tris(hydroxymethyl)-aminomethane, tricine, bicine, N-tris (hydroxymethyl )methyl-2-aminoethanesulfonic acid and others readily apparent to one skilled in the art.

In the practice of the sandwich assay of this invention, antibodies are immobilized in a suitable fashion on water-insoluble, particulate carrier materials formed from particulate organisms, polymers, glass, ceramics, natural or synthetic resins, diatomaceous earth or magnetizable particles. These particles are preferably spherical in shape and have an average diameter of from 0.01 to 10 μm, preferably from 0.1 to 10 μm, and most preferably of from 0.5 to 5 μm, although the structural and spatial configurations are not critical as long as they are not entrapped within the membrane (described below). The average diameter may be determined in relation to the pore size of the membrane so that the particles are retained on the membrane surface (as described below). The resulting water-insoluble reagent of antibody and particle is used in the method of this invention.

The antibodies can be affixed to particulate carrier materials by physical or chemical means, including adsorption or covalent reaction with reactive groups on the surface of the materials. Covalent attachment is preferred. Preferred polymeric particles have suitable surface reactive groups for covalently attaching the antibody molecules thereto. The density of the antibody molecules on the carrier materials may vary depending upon the composition of the materials, their size and the properties of the antibody, but sufficient density needed for adequate sensitivity in the assay can be readily determined by one skilled in the art. The antibodies can be modified appropriately for attachment if necessary.

Covalent attachment of antibody is usually accomplished using surface reactive groups which are capable of reacting directly or indirectly (that is through linkages, such as proteins or avidin-biotin) with free amine or sulfhydryl groups of the antibody. Such surface reactive groups include, but are not limited to, carboxy, epoxy, aldehyde, active halo atoms, activated 2-substituted ethylsulfonyl, vinylsulfonyl and other groups known in the art. The following discussion regarding preferred embodiments is for exemplification only, and is not meant to be limiting.

Particularly useful polymeric particles include those described in EP-A-0 323 692. Such particles are generally water-insoluble latex particles having an average particle size greater than 0.01 micrometers. They are composed of polymers prepared from one or more ethylenically unsaturated polymerizable monomers at least one of which has active halo atoms or activated 2-substituted ethylsulfonyl or vinylsulfonyl groups.

One or more of the monomers described above can be polymerized individually or in combination to form homo- or copolymers. Alternatively, and preferably, one or more of them are copolymerized with at least one other ethylenically unsaturated polymerizable monomer. Generally such monomers provide various desirable properties such as hydrophobicity, dispersibility or other features. Particularly useful comonomers are described in EP-A-0 323 692.

Representative useful polymers include the following: poly(m & p-chloromethylstyrene), poly(styrene-co-m & p-chloromethylstyrene-co-2-hydroxyethyl acrylate) (67:30:3 molar ratio), poly[styrene-co-m & p-(2-chloroethylsulfonylmethyl)-styrene] (96:4 molar ratio), poly{styreneco-N-[ m & p-(2-chloroethylsulfonylmethyl)phenyl]acrylamide) (99.3:0.7 molar ratio), poly(m & p-chloromethylstyrene-co-methacrylicacid)(95:5, 98:2 and 99.8:0.2 molar ratio), poly[styrene-co-m & p-(2-chloroethylsulfonylmethyl )styrene-co-methacrylic acid](93.5:4.5:2 molar ratio), poly{styrene-co-N-[m & p-(2-chloroethylsulfonylmethyl)phenyl]-acrylamide-co-methacrylic acid}(97.3:0.7:2 molar ratio), and poly(styrene-co-m & p-chloromethylstyrene)(70:30 molar ratio).

Procedures for attachment of antibodies to particles are well known in the art. Representative procedures are described, for example, in EP-A-0 323 692, US-A-3,925,157, US-A-4,181,636, US-A-4,703,018 and other references too numerous to mention. In general, the antibodies are mixed with the particles under suitable conditions depending upon the attachment form (absorption, covalent or use of linking groups). A worker skilled in the art would readily determine the appropriate conditions from the art as well as exemplary teachings presented herein. For example, for attachment to particles having reactive halo atoms, activated 2-substituted ethylsulfonyl or vinylsulfonyl groups, the antibodies are generally mixed with the particles for up to 24 hours at a temperature of from 20° to 40° C in a suspension buffered at a pH of from 7 to 10. If carboxy groups are used for reaction, the well known carbodiimide activators can be used, as well as carbamoylonium activators such as those described in EP-A-0 308 235. Antibodies can be absorbed onto particles by incubating particles and antibodies in suspension at suitable temperature for several hours.

An important aspect of the present invention is the use of a water-insoluble microporous membrane to which the reagent (antibody-particle) described above is affixed to form a water-insoluble article. This membrane has first and second outer surfaces, is inert to any chemical or biological reactions, and is generally composed of one or more natural or synthetic substances which have sufficient integrity for affixing the reagent and porosity for suitable filtration during the assay (generally an average pore size of from 0.5 to 10 $\mu$m). Useful membrane materials include, but are not limited to, natural or synthetic polymeric mats or fabrics, sintered glass, membranes or filters of glass or polymeric films or fibers, porous ceramic or cellulosic materials, porous particulate structures composed of beads bound together with an adhesive or binder material. The membranes are generally flat, but some irregularities in the surfaces are acceptable, as well as some curvature if it is desired. One skilled in the art can readily determine many commerically available materials or design others which are useful in this invention. Particularly useful materials are treated or untreated polyamide (particularly, nylon) microporous membranes, such as those commercially available from Pall Corp.

Generally, the microporous membrane has an average pore size of from 0.5 to 10 $\mu$meters. The membrane has sufficient porosity to quickly drain away fluid and uncomplexed materials encountered during an assay. Such uncomplexed materials include uncomplexed antigen, antibodies, cellular debris and other nonparticulate extraneous matter from a biological specimen. Therefore, the pore size of the membrane must be such that the noted materials will pass through the porous substrate while retaining the particulate reagent and the resulting complex with antigen.

The reagent is substantially on the surface of the membrane, meaning that less than 5% (by weight), and preferably, less than 1% (by weight) of the reagent is entrapped within the membrane. By "entrapped" is meant that the entire reagent particle is within a pore of the membrane. This does not mean that reagent particles cannot be partially embedded in pores at or near the outer surface. Unintentionally, the microporous membrane may have some reagent partially embedded within its surface pores, but not a substantial amount like that taught in EP-A-0 200 381 and WO-A-87/03690.

If desired, the membrane can be coated with proteins (such as casein or succinylated casein), as known in the art to reduce nonspecific interactions, or by surfactants to promote rapid filtration, or other optional materials which may facilitate the assay.

The particulate reagent described above is affixed to the surface of the membrane in any suitable manner as long as the antibodies are available for reaction with antigen in the contacting specimen. Generally, the reagent is affixed such that moderate mechanical disturbance during manufacture and handling does not loosen it. Moreover, the reagent generally does not come off the membrane during an assay. For example, the reagent can be affixed mechanically by coating, spotting or spraying and held thereon by hydrophobic bonding among molecules as well as between molecules and membrane. It can also be covalently affixed by suitable chemical reaction. Generally, the reagent is dried before use in an assay although drying is not critical.

It is particularly useful that the reagent be immobilized on the membrane in admixture with a hydrophilic, neutral or positively-charged binder material. The amount of binder material is generally less than 20% based on the total weight of reagent.

Particularly useful binders include, but are not limited to, vinylpyrrolidone polymers, acrylamide polymers, and polymers containing quaternary salts and others known in the art which are either neutral in charge or positively charged. By "neutral in charge" is meant that either the polymer has no charges, or has both negative and positive charges which provide a net zero charge in the molecule. The polymers can be homo- or copolymers, and used singly or in combination. Acrylamide polymers are also defined to include methacrylamide polymers.

Useful positively-charged polymeric binders include mordants described in US-A-4,069,017 and references mentioned therein, provided that the mordants are sufficiently water-soluble. Sufficient water-solubility is generally provided by having at least 50% by weight of the polymer be composed of monomers containing the positively-charged groups.

The particulate reagent can be affixed to a membrane surface in one or more discrete zones of the surface, each zone representing less than the total area of that surface. Alternatively, the reagent can be affixed to the entire membrane surface area.

The water-insoluble article thusly prepared can be used in an assay without other equipment or test containers (for example, as a hand-held article through which fluid is drained into container). Preferably however, it is disposed or mounted in a water-insoluble test device having a water-insoluble frame or structure for holding the membrane. Many forms of test devices are known in the art. Representative useful devices are those described in US-A-3,825,410, US-A-3,888,629, US-A-3,970,429 and US-A-4,446,232. Particularly useful devices are those marketed as part of Surecell™ test kits (Eastman Kodak Co.) for detection of antigenic materials. Such devices are described in EP-A-0 308 231 and EP-A-0 321 261.

More specifically, the preferred test device comprises a water-insoluble shell having one or more test wells therein, each of which can accommodate a sample of a biological specimen and appropriate reagents.

The shell can be prepared from any useful water-insoluble material such as glass, polymeric materials, ceramics, fibrous materials, cellulosic materials and other materials known in the art.

In a preferred embodiment, the test device has three test wells designed for providing a specimen test result and positive and negative control results. Each test well has a microporous membrane mounted therein, and at least one of the test wells has a membrane with the particulate reagent affixed thereto. Other variations of useful test devices would be within the purview of a worker of ordinary skill in the art.

Generally, the method of this invention can be carried out by contacting the membrane having the particulate reagent affixed thereto with an aqueous specimen suspected of containing extracted antigen from one or more serotypes of any of B. intermedius, B. gingivalis or A. actinomycetemcomitans. If antigen is present, an immunological complex is formed between antigen and antibodies and bound to the membrane.

At some point in the assay, either before, after or simultaneously with the formation of a complex between antigen and the antibodies of the particulate reagent, the antigen also forms an immunological complex with a second antibody to one or more serotypes of the organism which is water-soluble and detectably labeled. By "detectably labeled" is meant that the antibody has a moiety covalently attached thereto or otherwise associated therewith which can be directly or indirectly detected visually or by using spectrophotometer, radiometric or other suitable equipment and procedures. Indirect detection can be accomplished by reacting the moiety on the antibody one or more times with suitable reagents to render the resulting product directly detectable.

Useful labels are well known and include enzymes, radioistopes, specific binding materials such as biotin, avidin, lectins, sugars, and other materials readily apparent to one skilled in the art which can be detected in some manner. Preferably, the label is biotin, an enzyme or a radioisotope. Preferably, this contact with the water-soluble antibody occurs after complex formation with the insolubilized antibody on the membrane. The resulting bound complex is thus formed of antigen and two antibodies directed to different epitopic sites, one antibody insolubilized, the second antibody being labeled for detection.

The methods for preparation of labeled antibodies are well known. Enzymes, such as peroxidase, glucose oxidase, alkaline phosphatase, urease, $\beta$-galactosidase and glucosidase are particularly useful labels. Peroxidase is a most preferred label.

Simultaneously or subsequently to the contact of the labeled antibody with the antigen, uncomplexed materials are separated from the complex bound to the membrane by washing the uncomplexed materials through the membrane. There may be sufficient fluid in the biological specimen to wash the materials through the membrane, but generally, one or more separate wash fluids are applied to accomplish separation. Wash fluids are well known in the art, and include distilled water or buffered solutions of nonionic or anionic surfactants. A particularly useful wash fluid includes sodium decyl sulfate or sodium dodecyl sulfate.

Detection of the sandwich complex on the membrane is accomplished using standard detection equipment and procedures. They will vary with the particular label as one skilled in the art would readily perceive. Where the label is an enzyme, the bound complex is further contacted with a composition which will provide a dye (chromogen or fluorogen) in the presence of the enzyme. Such as composition generally includes one or more reagents which are substrates for the enzyme. Such reagents are known to one skilled in the art.

Where peroxidase is the label, a number of suitable dye-forming compositions are known comprising a substrate or substrate-forming reactants as well as dye-forming reactants. The substrate itself can be a dye-forming compound, such as benzidine, tetramethylbenzidine or other benzidine derivatives, 2,2'-azino-di-(3-ethylbenzthiazolone-6-sulfonic acid), phenol red, o-phenylenediamine, pyrogallol, 4-aminoantipyrine, bromopyrogallol red and others known in the art. Alternatively, a hydrogen donor and an electron acceptor can be combined to provide a detectable species as is known in the art.

Preferably, the dye-forming composition includes a leuco dye which provides a dye in the presence of hydrogen peroxide and peroxidase (for example, a triarylimidazole leuco dye as described in US-A-4,089,747 or a triarylmethane leuco dye as described in US-A-4,670,385). A preferred dye-providing composition is illustrated in the examples below.

Once a dye has been formed in the presence of the insoluble complex, it can be evaluated visually or by using spectrophotometric equipment to determine if the assay indicates the presence of antigen in the specimen. Both positive and negative control tests may be desirably carried out with the specimen test. Appropriate reagents could be used for each control test to give the desired result.

In one embodiment, where the label is biotin or avidin, detection can be achieved by reacting the label with the corresponding receptor (that is, biotin as the label reacted with avidin). The corresponding receptor can be suitable labeled with a radioisotope or enzyme to render the resulting complex detectable. For example, if biotin is the label, it can be reacted with a conjugate of an enzyme and avidin. The enzyme can produce a detectable dye in the presence of appropriate reagents.

A preferred method for the determination of one or more serotypes of any of the microorganisms Bacteroides intermedius, Bacteroides gingivalis or Actinobacillus actinomycetemcomitans comprises the steps of:

A. extracting antigen from one or more serotypes of any of the microorganisms Bacteroides intermedius, Bacteroides gingivalis or Actinobacillus actinomycetemcomitans present in a biological specimen,

B. contacting the extracted antigen with a microporous membrane in a disposable test device, the membrane having affixed thereto a water-insoluble reagent comprising polymeric particles having covalently attached thereto antibodies to the extracted antigen, the reagent being substantially all on the surface of the membrane,

to form a water-insoluble immunological complex between the extracted antigen and the antibodies present on the membrane,

C. contacting the water-insoluble complex with an enzyme-labeled, water-soluble antibody to the antigen so as to form an enzyme-labeled, water-insoluble immunological complex of both the labeled and water-insoluble antibodies with the antigen on the membrane,

D. washing uncomplexed materials through the membrane, and

E. contacting the enzyme-labeled complex on the membrane with a composition which provides a dye in the presence of the enzyme to indicate the presence of the antigen in the specimen.

The diagnostic test kit of this invention includes the article of this invention as well as detectably labeled antibodies directed to the particular serotype or combination of serotypes that the article is designed for. In some kits, the articles and labeled antibodies are used to detect

any serotype of the microorganism indiscriminately. Where the article has separate reaction zones for detecting various serotypes of the same microorganism, the kit can include the corresponding labeled antibodies. These kit components can be packaged, individually or together, in a suitable manner and included in a carrier of some type which can be compartmentalized to hold the article (alone or in a test device) and vials or bottles of reagents. In addition, it can also include one or more of the following which are optionally useful in carrying out the screening method: dye-forming composition, extraction reagents, wash solutions, diluents, and other reagents known to one skilled in the art for a given assay format. Reagents can be provided in dry form or in appropriate solutions. Non-reactive components of the kit can include instructions, mixing vessels, stirring means, pipettes and the like.

As noted above, the method of this invention can be used to detect any or all serotypes of the same microorganism. In one embodiment, each serotype can be detected individually by having separate test devices for each serotype. In addition, each serotype can be detected in a specific region of a single membrane in a test device. It may also be possible to differentiate one serotype (for example, serotype B) from the other two serotypes. Alternatively, where there is no need to differentiate serotypes, all serotypes of a given microorganism present in a specimen can be effectively detected.

The following preparation of polyclonal antibodies is a preferred method for their preparation.

The bacterial strains were supplied as viable cultures by Homer S. Reynolds (SUNY, Buffalo, New York School of Dentistry). Isolates were anaerobically cultured on CDC anaerobic plates (BBL Laboratories, Baltimore, Maryland) supplemented with hemin (5 μg/ml) and menadione (0.5 μg/ml).

The plates were then incubated for 24-48 hours at 37° C in an anaerobic chamber. Frozen stocks were prepared for each strain by harvesting the colonies with an inoculating loop, preparing a cell suspension in skim milk and freezing the suspension at -70° C. Viability of the frozen stock was tested by plating a portion of the frozen stock onto CDC anaerobic plates and incubating as described above. Once viability was determined, fresh isolates for each strain could be obtained in a similar manner. Cell suspensions were prepared by harvesting colonies with an inoculating loop, placing the loop in phosphate buffered saline solution (pH 7.5, 1 ml) and vortexing vigorously for one minute.

Concentrations of cell suspensions were determined spectrophotometrically by measuring turbidity at 620 nm. Appropriate dilutions were prepared to yield an optical density in the range of 0.1 to 1. Concentrations were determined using a 1 McFarland standard wherein $OD_{620}$ = 0.180, corresponds to 3 x $10^8$ cells/ml.

Ten New Zealand white rabbits were intravenously injected with 0.5 ml of a phosphate buffered saline solution (pH 7.3) solution of the respective immunogen containing 5 x $10^8$ whole viable cells per ml. The injections were given following the method shown in McCarty and Lancefield (J.Exp.Med., 102, pp. 1-28, 1955).

Two booster injections were given as follows: one week after the initial injection, each rabbit was injected again with the same amount of immunogen, and one additional week later, each rabbit was similarly injected.

Beginning with the fifteenth day after the initial injection, for a total of eleven additional weeks, each rabbit was given a booster injection of 1 ml of immunogen (5 x $10^8$ cells/ml) three times per week. The booster injections were spaced out every other day over each seven day period. Samples (2 ml) of antisera (for each serotype) were collected from the rabbits at various times to determine the antibody performance at those times. For example, antisera samples were taken at three, six and nine week intervals after the initial injection of immunogen. The rabbits were then sacrificed at thirteen weeks after the initial injection and the antisera was collected (100 ml per rabbit).

The final sera were then purified using ammonium sulfate precipitation. Saturated ammonium sulfate solution was added dropwise with stirring to each serum sample, cooled on ice until 45% saturation was achieved. After the addition, the mixture was stirred for an additional ten minutes, centrifuged and the supernatant discarded. The pellet was resuspended in a volume of phosphate buffered saline solution (pH 7.3) equal to the amount of the original sample being purified. The noted steps (addition of ammonium sulfate, centrifugation and resuspension of the pellet) were repeated. The mixture was then transferred to dialysis bags and dialyzed for fifteen hours at 4° C with stirring against phosphate buffered saline solution (pH 7.3). About 200-10,000 times excess of dialysis buffer was used. The dialysis was repeated with fresh buffer for an additional six hours. The solutions were removed from the dialysis bags and filtered through a 0.22 μmeter filter. A portion of the filtrate was diluted in the range of 1:10 to 1:100 in phosphate buffered saline solution, and the absorbance was read at 280 nm. The antibody concentration was determined according to the formula: $A_{280} \div$ [1.4 x (dilution)] = antibody conc.(mg/ml)

The antibody solutions were then diluted to 2-4 mg/ml with phosphate buffered saline solution (pH 7.3) and merthiolate (0.01 weight %) and stored at 4° C (for small amounts whereas large amounts were stored at -70° C).

The following examples are presented to illustrate the practice of the invention, and in no manner are they intended to limit its scope. All percentages are based on weight, unless otherwise indicated.

Example: 1 Sandwich Assay for All Three Serotypes of *Bacteroides intermedius*

This example demonstrates the method of this invention for the determination of all three serotypes of *B. intermedius* in a biological specimen by means of a sandwich assay.

Materials and Methods:

Surecell™ disposable test devices were used containing 5 μm LoProdyne™ nylon microporous membranes (Pall Corp.) incorporated into the test wells. The membranes had been pretreated with Fluorad™ FC 135 nonionic surfactant (0.05 g/m², 3M Co.).

A dye-providing composition was prepared to include 2-(4-hydroxy-3,5-dimethoxyphenyl)-4,5-bis-(4-methoxyphenyl)imidazole (0.008%), poly-(vinylpyrrolidone) (1%), sodium phosphate buffer (10 mmolar, pH 6.8), hydrogen peroxide (10 mmolar), 4'-hydroxyacetanilide electron transfer agent (2 mmolar) and diethylenetriaminepentaacetic acid chelating agent (10 μmolar).

Polyclonal antibodies directed against three serotypes of B. intermedius, serotype A (ATCC 25611), serotype B (NCTC 9336) and serotype C (ATCC 49046) were obtained by intravenous injection of rabbits as described above. IgG fractions were prepared by ammonium sulfate precipitation, and stored at 4°C in phosphate buffered saline solution (0.3-0.4% solution). NCTC is the National Collection of Type Cultures (London) and ATCC is the American Type Culture Collection (Rockville, Maryland).

The bacterial strains used to produce the antisera were supplied as viable cultures by H. S. Reynolds (School of Dentistry, SUNY, Buffalo). Isolates were subcultured onto anaerobic plates as described above. The antigens were extracted with sodium dodecyl sulfate (10%) for about one minute at room temperature.

Polymer-antibody reagents were prepared by covalently binding equal amounts of antibodies directed to each serotype of B. intermedius to polymeric particles of poly[styrene-co-m & p-(2-chloroethylsulfonylmethyl)styrene] (96:4 molar ratio) which had been made using the teaching of EP-A-0 323 692. Covalent attachment was achieved by adding the antibodies (56.5 μg in 67 μl of phosphate buffered saline solution) to a solution of borate buffer (659 μl, 0.05 molar, pH 8.5) in a test tube and mixed well. The polymeric particles (228 μl suspension containing 13.15% solids) were added to the antibody composition, and the resulting suspension was rotated end-over-end for 14-24 hours at room temperature to allow covalent attachment of antibodies to the particles. The suspension was then centrifuged at 2800 rpm for 10 minutes. The supernatant was discarded and the pellet was suspended in glycine buffer (1 ml, 0.01 molar, pH 8.5) containing merthiolate (0.01%). This procedure was repeated twice.

The final antibody/particle reagent composition contained the following:
antibody/particle reagent (1%), polyacrylamide binder (5%) and merthiolate preservative (0.01%) in glycine buffer (0.1 molar, pH 8.5). This reagent composition (2 μl) was then applied to a region of a LoProdyne™ nylon microporous membrane in the test devices and dried to form an article of the present invention.

Enzyme-antibody conjugates were prepared from serotypes A, B and C fractions, each conjugated to horseradish peroxidase using the procedure of Yoshitake et al (Eur. J. Biochem., 101, 395, 1979). The conjugate composition comprised the conjugates (5 μg of each per ml), casein (0.5%) and merthiolate (0.01%) in 3-(N-morpholino)-propanesulfonic acid buffer (0.1 molar, pH 7.5).

A wash solution comprised sodium decyl sulfate (18 g/l) in water.

Assay:

The extracted antigen (50 μl) containing antigens from serotypes A, B and C of B. intermedius were added to the test wells of the test devices described above containing the polymer-antibody reagents on the membranes. Sodium dodecyl sulfate solution (10%) was added to one test well of each device as negative controls.

The conjugate composition (50 μl) was added and the test devices were incubated at room temperature (18-25°C) for 5 minutes. The wash solution (240 μl) was added twice to the test wells. The dye-providing composition (50 μl) was added and the test devices incubated at room temperature for 2 minutes. The dye formed on the membranes was visually read and compared to a calibrated color chart having reflectance density values, then converted to transmittance density ($D_T$) using the Williams-Clapper transform (J. Opt. Soc. Amer., 43, 595, 1953).

The results of the assay are plotted in FIG. 1 as absolute $D_T$ vs. the logarithm of the absolute cell concentration. They show that the method of this invention is useful to detect all three serotypes of B. intermedius with good sensitivity. The detection minimum was about $10^4$ cells.

Example 2: Comparative Example Showing Effect on Cross-Reactivity in Assay for B. intermedius

This example illustrates the practice of this invention using a sandwich assay in comparison with a direct binding assay to detect B. intermedius, and evaluates the cross-reactivity with B. loescheii observed in both assay methods. The direct binding assay is identified as a Control method.

Sandwich Assay:

The bacterial strains to B. intermedius - (identified above) and B. loescheii (ATCC 15930) were obtained from H. S. Reynolds (identified above), and grown in cultures as described above. A sandwich assay was carried out to detect B. loescheii using the procedure described in Example 1.

The results of the assay are shown in FIG. 2 which is a graphical plot of absolute transmittance density ($D_T$) vs. the logarithm of the number of cells. The plot indicates that B. loescheii was not detected with the method of this invention. Thus, no cross-reactivity was observed with this microorganism by the B. intermedius antibodies.

Control Direct Binding Assay:

SurecellTM disposable test devices were used in this assay as well. The had 5 $\mu$m BiodyneTM - (Pall Corp.) membranes in the test wells, which membranes had been coated with ZonylTM FSN nonionic surfactant (0.05 g/m$^2$).

A wash solution comprised 3-cyclohexylamino-2-hydroxy-1-propanesulfonic acid (0.05 molar), EmcolTM CC-9 cationic surfactant (0.75%, Witco Chemical Co.) and merthiolate (0.01%). The solution pH was raised to 10 using sodium hydroxide (0.05 normal).

Suspensions of bacterial antigens were prepared in phosphate buffered saline solution. All other materials were like those used in the sandwich assay.

The extracted antigen solution (50 $\mu$l) containing antigens from both B. intermedius and B. loescheii was added to the test wells of the test devices, and allowed to bind to the membranes. Phosphate buffered saline solution was added to one test well of each device to provide negative controls.

The conjugate solution (50 $\mu$l) was added to all the test wells and the test devices were incubated at room temperature for 5 minutes. The wash solution (240 $\mu$l) was added twice to the test wells, followed by addition of the dye-providing composition (50 $\mu$l). After incubation at room temperature for 2 minutes, the dye on the membrane was visually observed and converted to transmittance density as described in Example 1.

The results of this Control method are shown in FIG. 3. It is clear that considerable cross-reactivity occurred between the B. intermedius antibodies and the B. loescheii antigens, especially at the higher antigen concentrations.

Example 3: Differentiation of Serotypes by Sandwich Assay

This example demonstrates how the present invention can be used to differentiate the serotypes of the organism B. intermedius using a single disposable test device.

Materials and Methods:

Polyclonal antibodies directed to serotypes B and C of B. intermedius were prepared as described in Example 1.

Polymer-antibody reagents were prepared as described in Example 1 using serotype B and C antibodies. The reagent containing serotype B antibodies (2 $\mu$l) was added to the wells of a test device described for the sandwich assay in Example 2. The reagent containing serotype C antibodies (2 $\mu$l) were added to the test wells of a test device like that used in the direct binding assay described in Example 2.

Enzyme-antibody conjugates were prepared from the serotype B and C antibodies, through conjugation to horseradish peroxidase using the Yositake et al procedure identified in Example 1. Each conjugate composition contained the conjugate (9 $\mu$g of respective antibody per ml of solution), casein (0.5%) and merthiolate (0.01%) in 3-(N-morpholino)propanesulfonic acid buffer (0.1 molar, pH 7.5).

Bacterial strains corresponding to serotypes A, B and C were extracted using sodium dodecyl sulfate (0.05%) for about one minute at room temperature.

Assay:

Part A: The extracted antigen solution containing antigens from serotypes A, B and C were added to the wells of the test device containing serotype B polymer-antibody reagent. Sodium dodecyl sulfate (0.05%) was put in one test well of each device to provide negative controls.

The conjugate composition containing antibodies to serotypes B (50 $\mu$l) was added to the test wells and the device was incubated at room temperature for 5 minutes. The wash solution (240 $\mu$l) was added twice to the test wells, followed by addition of the dye-providing composition (50 $\mu$l). After incubation at room temperature for 2 minutes, the dye on the membrane was visually evaluated and converted to transmittance density as described above.

The results are shown in FIG. 4, and indicate that serotype B antibodies are specific to serotype B antigen in the sandwich assay of this invention.

Part B: The foregoing sandwich assay was repeated using a polymer-antibody reagent having antibodies directed to serotype C of B. intermedius. The results are shown in FIG. 5, and indicate that serotype C antibodies can be used to detect both serotype A and C antigens in the sandwich assay of this invention.

Example 4: Comparative Assays for All Three Serotypes of Actinobacillus actinomycetemcomitans

This example demonstrates the method of this invention for the determination of all three serotypes of A. actinomycetemcomitans in a biological specimen by means of a sandwich assay. It also demonstrates the greatly reduced cross-reactivity of the antibodies in this assay with Haemophilus paraphrophilus and Haemophilus parainfluenza over a direct binding assay in which the same antibodies show high cross-reactivity with the Haemophilus organisms.

Materials and Methods for Sandwich Assay:

The Surecell™ disposable test devices and a dye-providing composition were the same as those in Example 1.

Antiserum to serotype A of A. actinomycetemcomitans (identified as strain 75, ATCC 43717) was obtained from Dr. J. J. Zambon and H. S. Reynolds (SUNY, Buffalo, New York School of Dentistry). ATCC represents the American Type Culture Collection (Rockville, Maryland). IgG fractions were prepared by ammonium sulfate precipitation, and covalently bound to polymeric particles of poly-[styrene-co-m & p-(2-chloroethylsulfonyl-methyl)-styrene] (96:4 molar ratio) which had been made using the teaching of EP-A-323 692. Covalent attachment was achieved by adding the antibodies directed to serotype A (56.5 μg in 67 μl of phosphate buffered saline solution) to a solution of borate buffer (659 μl, 0.05 molar, pH 8.5) in a test tube and mixed well. The polymeric particles (228 μl suspension containing 13.15% solids) were added to the antibody composition, and the resulting suspension was rotated end-over-end for 14-24 hours at room temperature (generally 18-25° C) to allow covalent attachment of antibodies to the particles. The suspension was then centrifuged at 2800 rpm for 10 minutes. The supernatant was discarded and the pellet was suspended in glycine buffer (1 ml, 0.01 molar, pH 8.5) containing merthiolate (0.01%). This procedure was repeated twice.

The final antibody/particle reagent composition contained the following:
antibody/particle reagent (1%), polyacrylamide binder (5%) and merthiolate preservative (0.01%) in glycine buffer (0.1 molar, pH 8.5). This reagent composition was then applied to a region of a LoProdyne™ nylon microporous membrane in the test devices and dried to form an article of the present invention.

Enzyme-antibody conjugates were prepared from serotype A IgG fractions, conjugated to horseradish peroxidase using the procedure of Yoshitake et al (Eur. J. Biochem., 101, 395, 1979). The conjugate composition comprised the conjugate (2.5 μg/ml), casein (0.5%) and merthiolate (0.01%) in 3-(N-morpholino)propanesulfonic acid buffer (0.1 molar, pH 7.5).

A wash solution comprised sodium decyl sulfate (18 g/l) in water.

Bacterial strain 75, corresponding to serotype A of A. actinomycetemcomitans, Haemophilus paraphrophilus (ATCC 29241) and Haemophilus parainfluenza (NCTC 9796) were obtained from Dr. J. J. Zambon and H. S. Reynolds (SUNY, Buffalo School of Dentistry), and grown as described in by Zambon et al (Infect. Immun., 41(1), pp. 19-27, 1983). The antigens were extracted from these species using sodium dodecyl sulfate (0.05%) for about one minute at room temperature. NCTC represents the National Collection of Type Cultures (London).

Materials and Methods for Direct Binding Assay:

Surecell™ disposable test devices were used which contained 5 μm Biodyne™ nylon microporous membranes (Pall Corp.) coated with Zonyl™ FSN nonionic surfactant (0.05 g/m$^2$).

A wash solution comprised Emcol™ CC-9 cationic surfactant (0.75%, Witco Chemical Co.), merthiolate (0.01%) in 3-cyclohexylamino-2-hydroxy-1-propanesulfonic acid buffer (0.05 molar). The pH of the solution was raised to 10 by the addition of sodium hydroxide (0.05 Normal).

Suspensions of bacterial antigens to A. actinomycetemcomitans, Haemophilus paraphrophilus and Haemophilus parainfluenza were prepared in phosphate buffered saline solution. All other materials were the same as those for the sandwich assay.

Sandwich Assay:

The extracted antigen solutions (50 μl), containing antigens from Haemophilus paraphrophilus, Haemophilus parainfluenza and A. actinomycetemcomitans were added to separate test wells of test devices containing the serotype A antibody-particle reagent on the membranes. Sodium dodecyl sulfate (50 μl, 0.05%) was added to control test wells as a negative control.

The conjugate composition (50 μl) was added and the test devices were incubated at room temperature for 5 minutes. The wash solution (240 μl) was added twice to the top of the reagent on the membranes in the test wells. The dye-providing composition (50 μl) was added and the test devices were incubated for about 2 minutes at room temperature. The dye formed on the membranes was visually observed and compared to a calibrated color chart having reflectance density values, then converted to transmittance density ($D_T$) using the Williams-Clapper transform (J. Opt. Soc. Amer., 43, 595, 1953). The results of the assay are plotted graphically as absolute $D_T$ vs. the logarithm of the absolute cell concentration in FIG. 6. They indicate that antibodies to A. actinomycetemcomitans show negligible cross-reactivity with either Haemophilus species considered.

Control Direct Binding Assay:

The extracted antigen solutions (50 μl) containing the antigens of the three microorganisms were added to the test wells of separate disposable test devices and allowed to bind to the membranes in the test wells. Phosphate buffered saline solution (50 μl) was added to certain test wells used as negative controls.

The conjugate solution (50 μl) was added to all test wells of the devices followed by incubation at room temperature for 5 minutes. The wash solution was added to the test wells twice (240 μl) followed by addition of the dye-providing composition (50 μl). After incubation at room temperature for 2 minutes, the dye formed on the membranes was visually observed and converted to $D_T$ as described for the sandwich assay. The results are shown in FIG. 7, a plot of absolute $D_T$ vs. the logarithm of the absolute cell concentration. They indicate that considerable cross-reactivity occurred with both Haemophilus species, particularly at higher cell concentration.

Example 5: Differentiation of Serotypes by Sandwich Assay

This example demonstrates the practice of the present invention can be used to differentiate between serotypes of Actinobacillus actinomycetemcomitans using a single disposable test device. Serotype A antibody can be used to distinguish between serotype A antigen and serotype B and C antigens. Serotypes B antibody can be used to distinguish between serotype B and C antigens versus serotype A antigen.

Materials and Methods:

SurecellTM test devices and antibody-particle reagents were used like those prepared and used in Example 4 (sandwich assay). Enzyme-antibody conjugates were prepared from serotype A and B antibodies according to the procedures of Example 4. All other compositions were similar to those in Example 4 also.

Assay:

The antibody-particle reagent for each of serotypes A and B (2 μl each) were added to separate SurecellTM test devices.

Antigens from serotypes A, B and C were extracted as described in Example 4 for the sandwich assay, and the solutions (50 μl) were added to all wells of the test devices. Sodium dodecyl sulfate (50 μl of a 0.05% solution) was added to certain test wells as a negative control.

Enzyme-antibody conjugate to serotype A (50 μl) was added to test devices containing serotype A antibody-particle reagent and the conjugate to serotype B (50 μl) was added to other test devices containing serotype B antibody-particle reagent. All test devices were then incubated at room temperature for 5 minutes.

The wash solution (240 μl) was added twice to the test wells, followed by addition of the dye-providing composition (50 μl). After incubation at room temperature for 2 minutes, the dye formed on the membrane was visually observed and converted to $D_T$ as described in Example 4.

The results are shown in FIG. 8 as a plot of the difference (Δ) in $D_T$ between the sample and negative control wells vs. the logarithm of the number of cells. In FIG. 9, absolute $D_T$ is plotted vs. the logarithm of the absolute cell concentration.

The results of FIG. 8 indicate that serotype A antibody can be used to distinguish serotype A antigens from either serotype B or C antigens of A. actinomycetemcomitans in the range of about $10^3$ to about $10^5$ cells. The results of FIG. 9 show that serotype B antibody can be used to distinguish serotype B and C antigens from serotype A antigen in the range of about $10^3$ to about a $10^6$ cells.

Example 6: Sandwich Assay for All Three Serotypes of Bacteroides gingivalis

This example demonstrates the method of this invention for the determination of all three serotypes of Bacteroides gingivalis in a biological specimen by means of a sandwich assay.

Materials:

The SurecellTM disposable test devices and a dye-providing composition were the same as those used in Example 1.

Polyclonal antisera directed against three serotypes of Bacteroides gingivalis (serotype A, ATCC 33277, serotype B, ATCC 53978 and serotype C, ATCC 53977) were obtained by intravenous injection of rabbits as described above. IgG fractions were prepared by ammonium sulfate precipitation and stored in phosphate buffered saline solution (0.3-0.4%) at either 4°C for small quantities or at -70°C for larger quantities.

The bacterial strains used to produce the antisera were supplied as viable cultures by H.S. Reynolds (School of Dentistry, SUNY, Buffalo, New York). Isolates were subcultured onto anaerobic plates as described above. The antigens were extracted with sodium dodecyl sulfate (10%) for one minute at room temperature (about 18-25 °C).

Polymer-antibody reagents were prepared by covalently binding equal amounts of antibodies directed to each serotype of Bacteroides gingivalis to polymeric particles of poly[styrene-co-m & p-(2-chloroethylsulfonylmethyl)styrene] (96:4 molar ratio) which had been made using the teaching of EP-A-0 323 692. Covalent attachment was achieved by adding the antibodies (56.5 μg in 67 μl of phosphate buffered saline solution) to a solution of borate buffer (659 μl, 0.05 molar, pH 8.5) in a test tube and mixed well. The polymeric particles (228 μl suspension containing 13.15% solids) were added to the antibody composition, and the resulting suspension was rotated end-over-end for 14-24 hours at room temperature to allow covalent attachment of antibodies to the particles. The suspension was then centrifuged at 2800 rpm for 10 minutes. The supernatant was discarded and the pellet was suspended in glycine buffer (1 ml, 0.01 molar, pH 8.5) containing merthiolate preservative (0.01%). This procedure was repeated twice.

The antibody-particle reagent composition contained the following: antibody/particle reagent (1%), polyacrylamide binder (5%) and merthiolate (0.01%) in glycine buffer (0.1 molar, pH 8.5). This reagent composition (2 μl) was then applied to a region of a LoProdyne™ nylon microporous membrane in the test devices and dried to form an article of the present invention.

Enzyme-antibody conjugates were prepared from serotypes A, B and C fractions, each conjugated to horseradish peroxidase using the procedure of Yoshitake et al (Eur. J. Biochem., 101, 395, 1979). The conjugate composition comprised the conjugates (5 μg of each per ml), casein (0.5%) and merthiolate (0.01%) in 3-(N-morpholino)-propanesulfonic acid buffer (0.1 molar, pH 7.5).

A wash solution comprised sodium decyl sulfate (18 g/l) in water.

Assay:

An extracted antigen solution (50 μl) containing antigens from serotypes A, B and C of Bacteroides gingivalis was added to the test wells of the test devices described above containing the polymer-antibody reagents on the membranes. Sodium dodecyl sulfate was added to one well of each device to provide a negative control.

The conjugate composition (50 μl) was added and the test devices were incubated at room temperature for 5 minutes. The wash solution (240 μl) was added twice to the test wells. The dye-providing composition (50 μl) was then added and the test devices which were incubated at room temperature for 2 minutes. The dye formed on the membranes was visually read and compared to a calibrated color chart having reflectance density values, then converted to transmittance density ($D_T$) using the Williams-Clapper transform (J.Opt.Soc.Amer., 43, 595, 1953).

The results of the assay are plotted in FIG. 10 as absolute $D_T$ vs. the logarithm of the absolute cell concentration. They show that the method of this invention is useful to detect all three serotypes of Bacteroides(Prophyromonas) gingivalis with good sensitivity. The detection minimum was about $10^4$ cells.

Example 7: Sandwich Assay for Bacteroides Gingivalis Showing Reduced Cross-reactivity

This example demonstrates that the method of the Present invention does not detect Bacteroides species other than B. gingivalis. In other words there is no cross-reactivity with those other Bacteroides species.

The materials and assay were essentially like those described in Example 6. The bacterial strains, B. loescheii (ATCC 15930), B. asaccharalyticus (ATCC 25260) and B. intermedius (ATCC 25611) were subcultured and extracted as described for the organisms in Example 6.

Solutions (50 μl) of extracted antigen from the organisms noted above were added to separate test devices which contained particle-antibody reagents as described in Example 6 (that is, with antibodies to all three serotypes of B. gingivalis) on the membranes in the test wells. Sodium dodecyl sulfate (10%) solution was used as negative controls.

B. gingivalis antibodies labeled with perioxidase and dye-providing composition were added to see if any complex would form on the membranes. The results indicated that, for antigen concentrations up to $10^7$ cells, no reaction occurred. In contrast, Example 6 was repeated with B. gingivalis antigen and reaction occurred.

## Claims

1. A method for the determination of one or more serotypes of any of the microorganisms Bacteroides intermedius, Bacteroides gingivalis or Actinobacillus actinomycetemcomitans comprising the steps of:

   A. contacting an aqueous specimen suspected of containing antigen extracted from one or more serotypes of any of the microorganisms Bacteroides intermedius, Bacteroides gingivalis or Actinobacillus actinomycetemcomitans with a microporous membrane having thereon a water-insoluble reagent comprising water-insoluble particles having affixed thereto antibodies to the antigen, the reagent being substantially all on the surface of the membrane, to form a water-insoluble immunological complex between the antibodies and extracted antigen present in the specimen on the membrane,

B. prior to, simultaneously with or subsequent to the contact in step A, contacting the extracted antigen with a detectably labeled, water-soluble antibody to the antigen so as to form a labeled water-insoluble immunological complex of both the labeled and water-insoluble antibodies with extracted antigen in the specimen on the membrane,

C. simultaneously with or subsequently to the contacting in step B, separating the labeled water-insoluble complex from uncomplexed materials by washing the uncomplexed materials through the microporous membrane, and

D. detecting the labeled complex on the membrane as an indication of the presence of the antigen in the specimen.

**2.** The method as claimed in claim 1 for the detection of a single serotype of the organism.

**3.** The method as claimed in claim 1 for the individual detection of all serotypes of the microorganism wherein each serotype is detected in a discrete zone of the membrane which is less than its total surface area using a water-insoluble reagent specific for each serotype.

**4.** The method as claimed in claim 1 for the indiscriminate detection of all serotypes of the organism in a single discrete zone of the membrane.

**5.** The method as claimed in any of claims 1 to 4 wherein the soluble antibodies are labeled with an enzyme, radioisotope or biotin.

**6.** The method as claimed in any of claims 1 to 5 for the detection of a lipopolysaccharide or capsule antigen of Bacteroides intermedius or Bacteriodes gingivalis.

**7.** A water-insoluble article comprising a microporous membrane, having affixed to at least one of its surfaces, a water-insoluble reagent comprising water-insoluble particles,
the article characterized wherein the particles have affixed thereto antibodies directed to at least one serotype of any of the microorganisms Bacteroides intermedius, Bacteroides gingivalis or Actinobacillus actinomycetemcomitans, the reagent being substantially all on the surface.

**8.** The article as claimed in claim 7 wherein the reagent is affixed to a discrete zone which is less than the total area of the surface of the membrane.

**9.** The article as claimed in either of claims 7 and 8 wherein less than 1%, by weight, of the reagent is entrapped by the membrane.

**10.** A test kit for the detection of one or more serotypes of any of the microorganisms Bacteroides intermedius, Bacteroides gingivalis or Actinbacillus actinomycetemcomitans comprising:

a. a water-insoluble article comprising a microporous membrane, having affixed to at least one of its surfaces, a water-insoluble reagent comprising water-insoluble particles, and

b. detectably labeled antibodies, the kit characterized wherein the particles have affixed thereto antibodies directed to at least one serotype of any of the microorganisms Bacteroides intermedius, Bacteroides gingivalis or Actinobacillus actinomycemtemcomitans, the detectably labeled antibodies are directed to the same serotype of Bacteroides intermedius, Bacteroides gingivalis or Actinobacillus actinomycetemcomitans as the antibodies affixed to the water-insoluble particles, and the reagent is substantially all on the membrane surface.

**11.** The invention as claimed in any of claims 1 to 10 wherein the antibodies are covalently attached to the polymeric particles.

**12.** The invention as claimed in any of claims 1 to 11 wherein the membrane has a pore size of from 0.5 to 10 $\mu$meters and the particles have an average diameter of from 0.1 to 10 $\mu$meters.

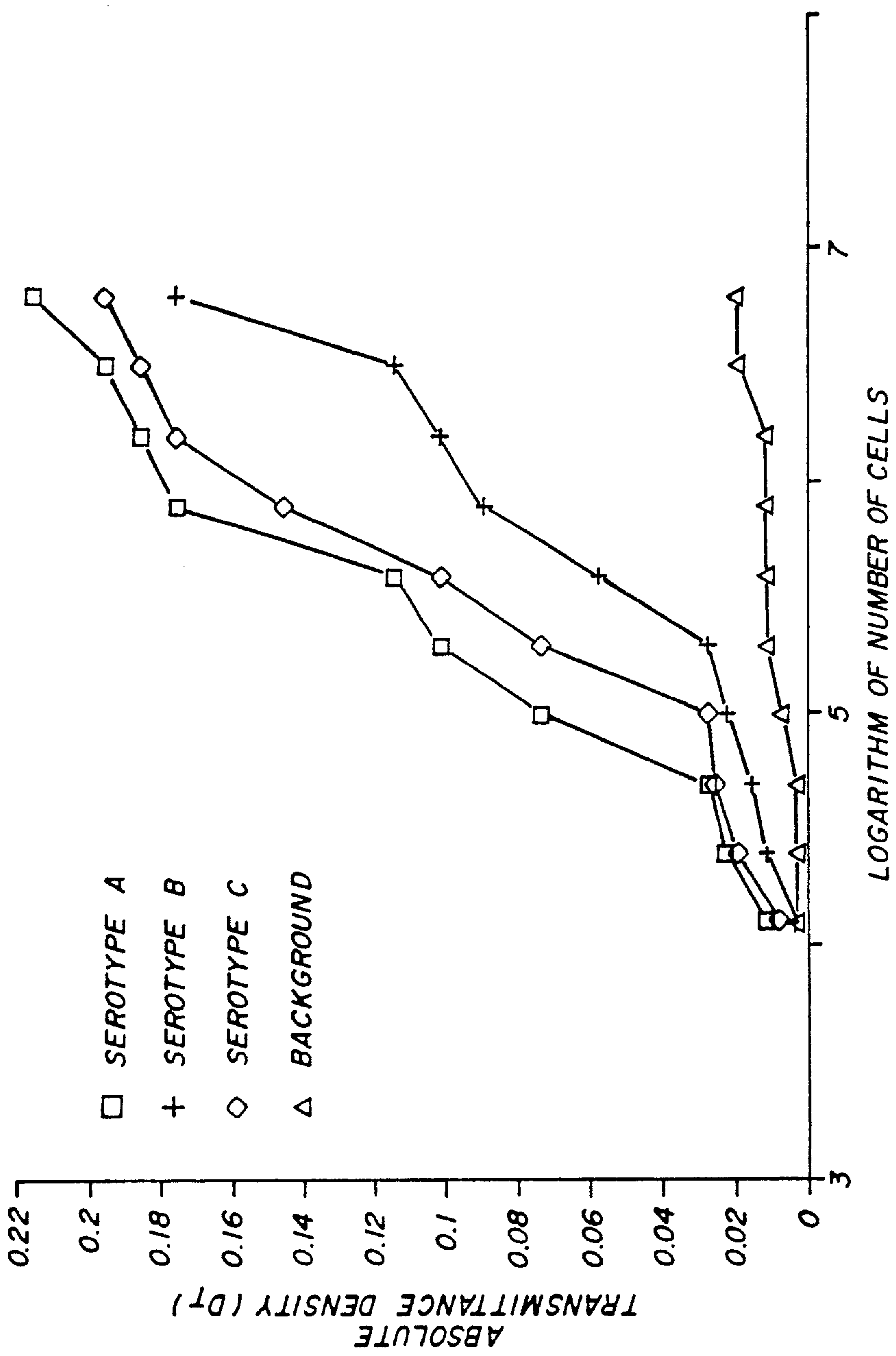
□ SEROTYPE A
+ SEROTYPE B
◇ SEROTYPE C
△ BACKGROUND
ABSOLUTE TRANSMITTANCE DENSITY ($D_T$)
0.22
0.2
0.18
0.16
0.14
0.12
0.1
0.08
0.06
0.04
0.02
0
3
5
7
LOGARITHM OF NUMBER OF CELLS

FIG. I

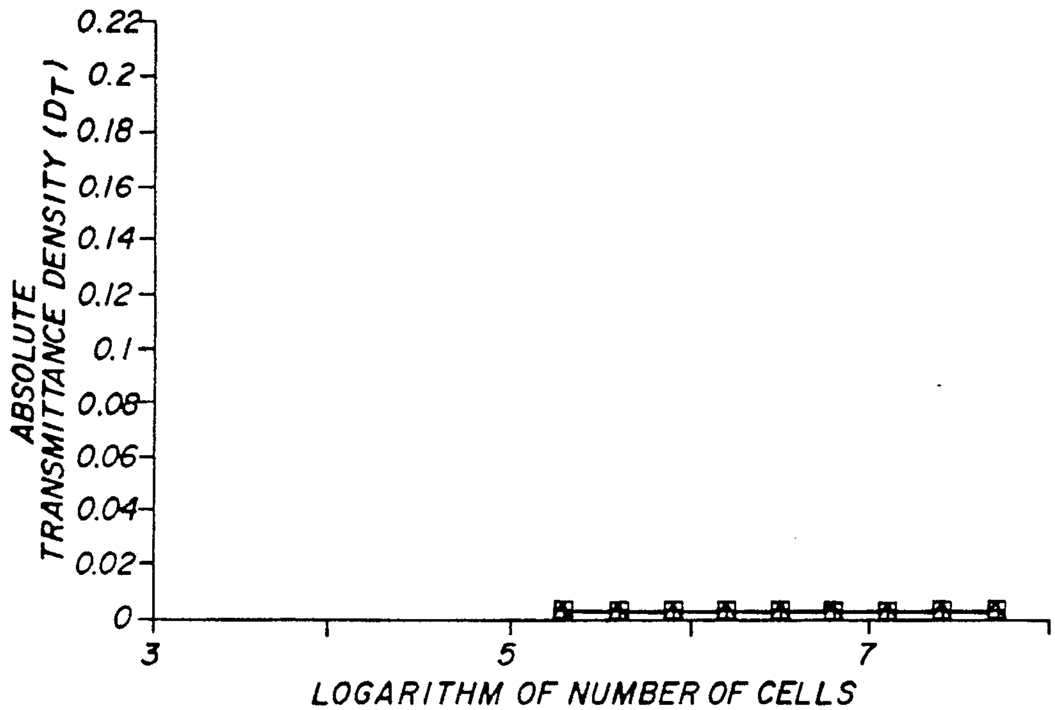
ABSOLUTE TRANSMITTANCE DENSITY ($D_T$)
0.22
0.2
0.18
0.16
0.14
0.12
0.1
0.08
0.06
0.04
0.02
0
3
5
7
LOGARITHM OF NUMBER OF CELLS

FIG. 2

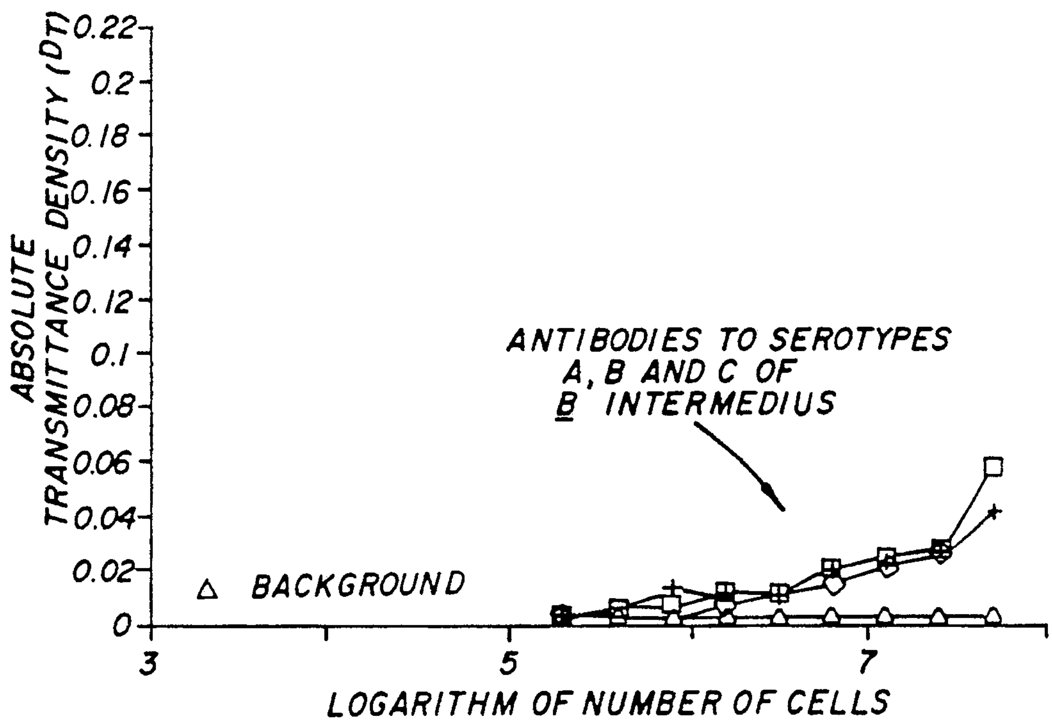
ABSOLUTE TRANSMITTANCE DENSITY ($D_T$)
0.22
0.2
0.18
0.16
0.14
0.12
0.1
0.08
0.06
0.04
0.02
0
3
5
7
ANTIBODIES TO SEROTYPES A, B AND C OF B INTERMEDIUS
△ BACKGROUND
LOGARITHM OF NUMBER OF CELLS

FIG. 3

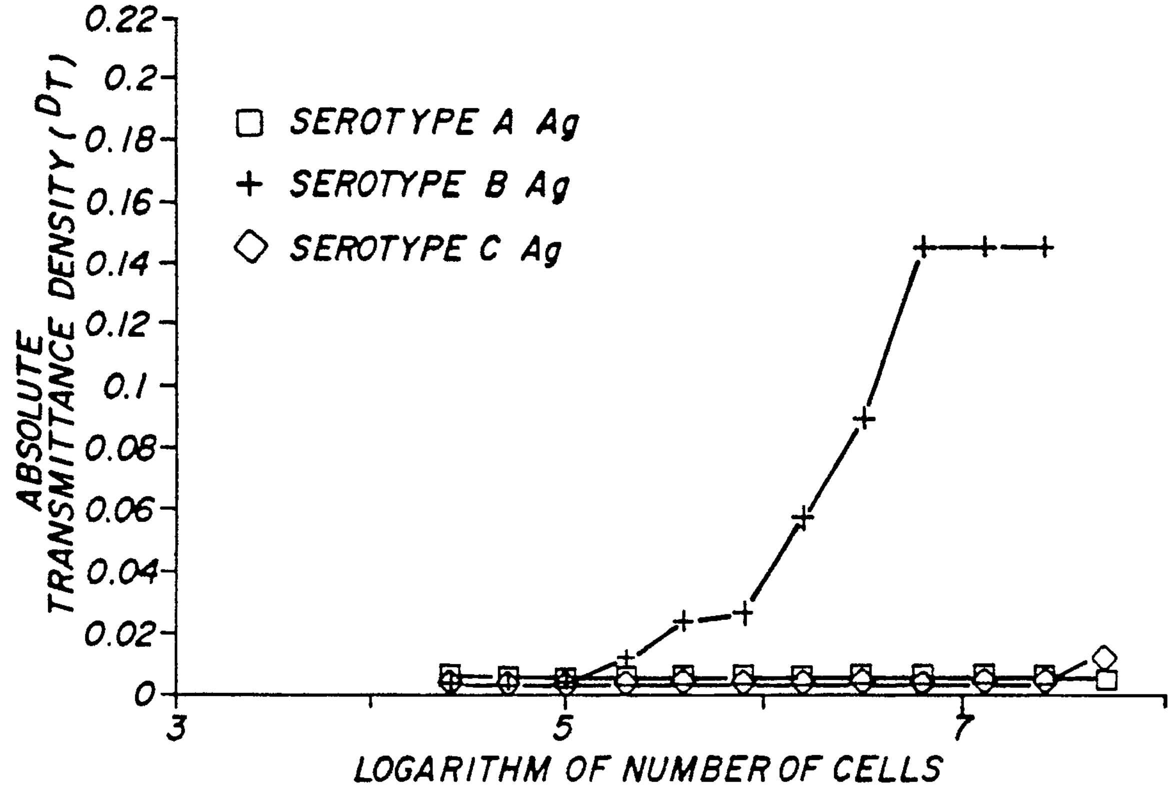
0.22
0.2
0.18
0.16
0.14
0.12
0.1
0.08
0.06
0.04
0.02
0
ABSOLUTE TRANSMITTANCE DENSITY ($D_T$)
SEROTYPE A Ag
SEROTYPE B Ag
SEROTYPE C Ag
3
5
7
LOGARITHM OF NUMBER OF CELLS

FIG. 4

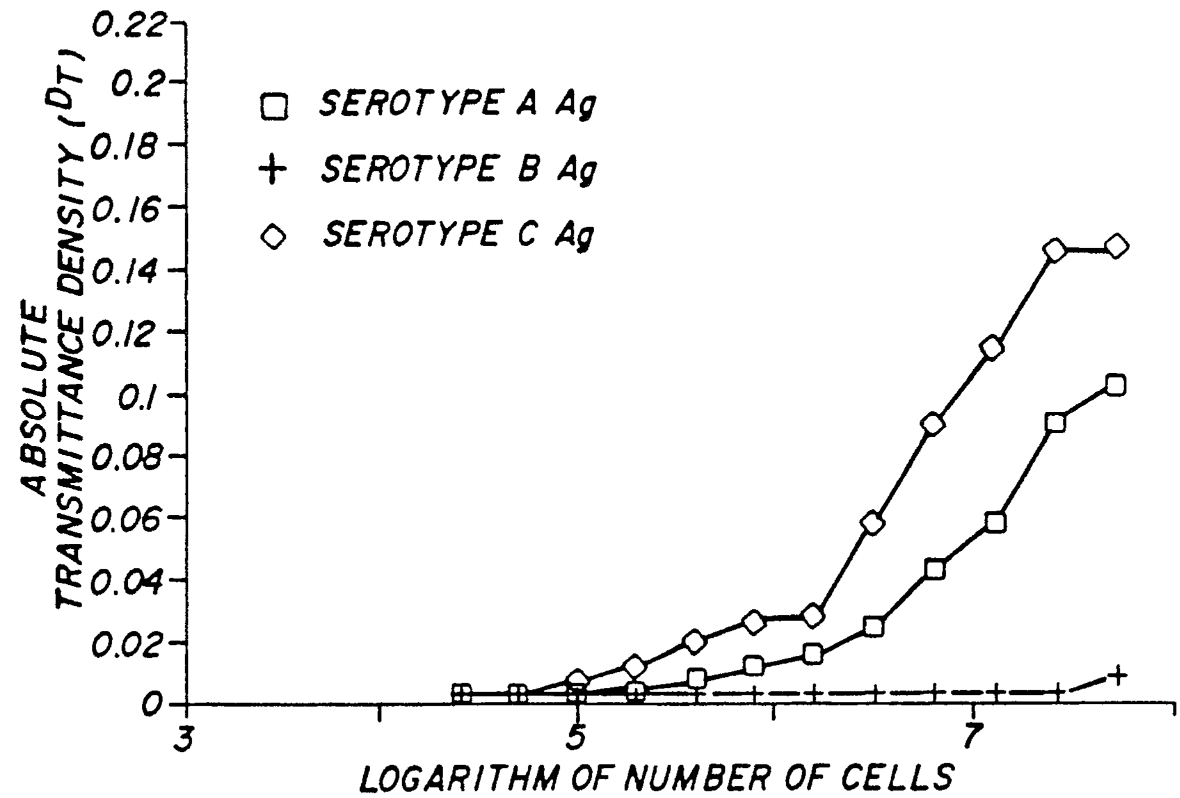
0.22
0.2
0.18
0.16
0.14
0.12
0.1
0.08
0.06
0.04
0.02
0
ABSOLUTE TRANSMITTANCE DENSITY ($D_T$)
SEROTYPE A Ag
SEROTYPE B Ag
SEROTYPE C Ag
3
5
7
LOGARITHM OF NUMBER OF CELLS

FIG. 5

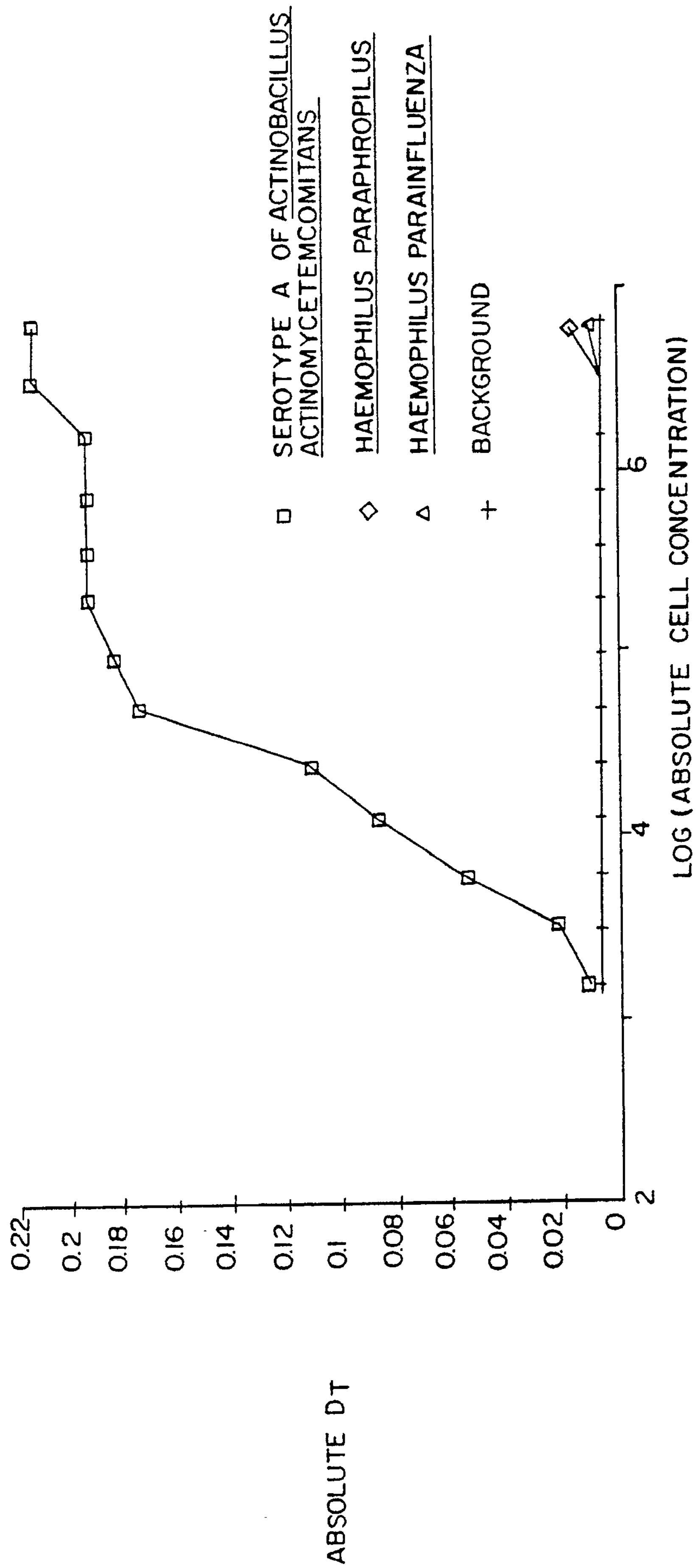
SEROTYPE A OF ACTINOBACILLUS ACTINOMYCETEMCOMITANS
HAEMOPHILUS PARAPHROPILUS
HAEMOPHILUS PARAINFLUENZA
BACKGROUND
ABSOLUTE DT
0.22
0.2
0.18
0.16
0.14
0.12
0.1
0.08
0.06
0.04
0.02
0
2
4
6
LOG (ABSOLUTE CELL CONCENTRATION)

FIG. 6

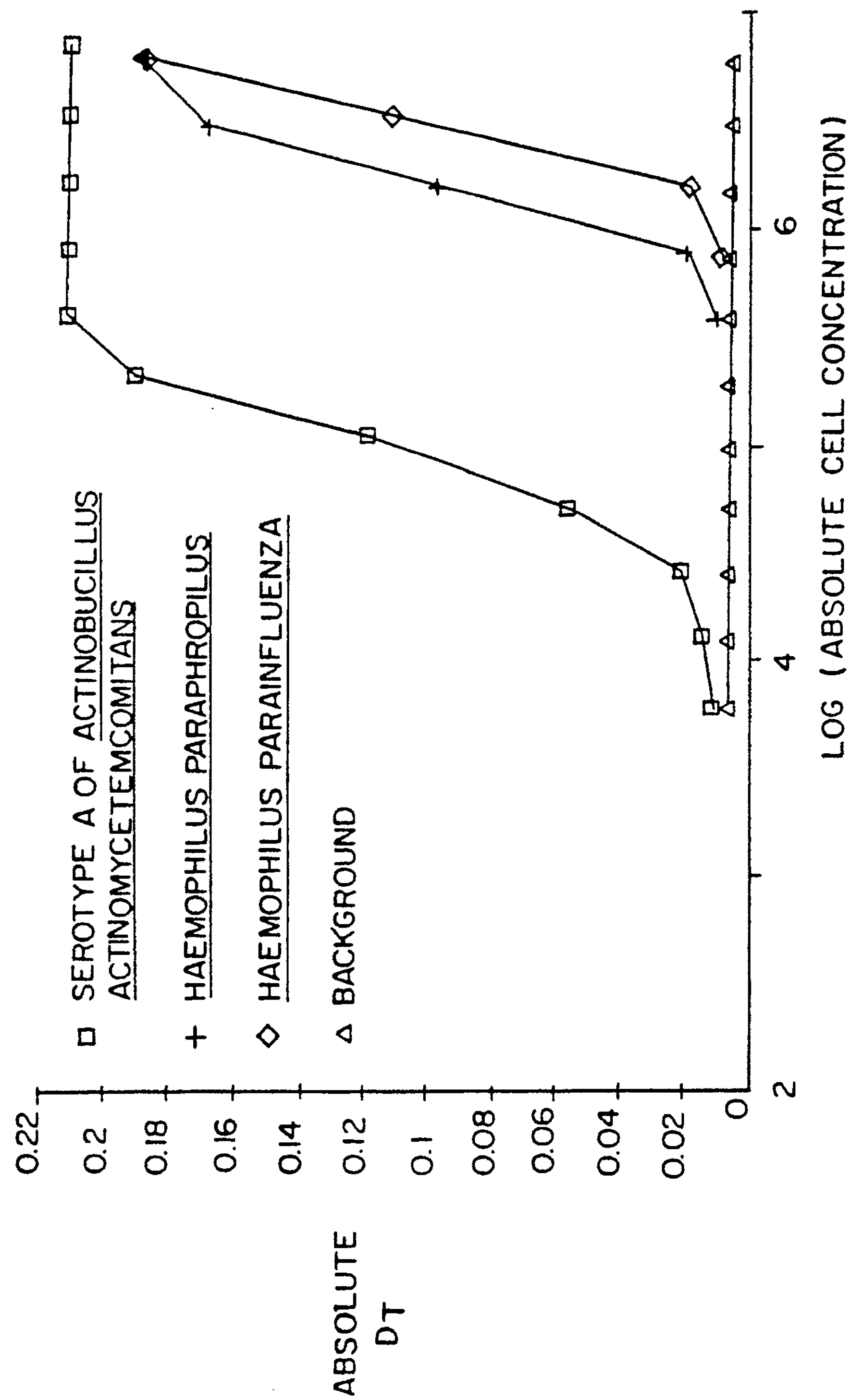
SEROTYPE A OF ACTINOBUCILLUS ACTINOMYCETEMCOMITANS
HAEMOPHILUS PARAPHROPILUS
HAEMOPHILUS PARAINFLUENZA
BACKGROUND
ABSOLUTE
DT
0.22
0.2
0.18
0.16
0.14
0.12
0.1
0.08
0.06
0.04
0.02
0
2
4
6
LOG (ABSOLUTE CELL CONCENTRATION)

FIG. 7

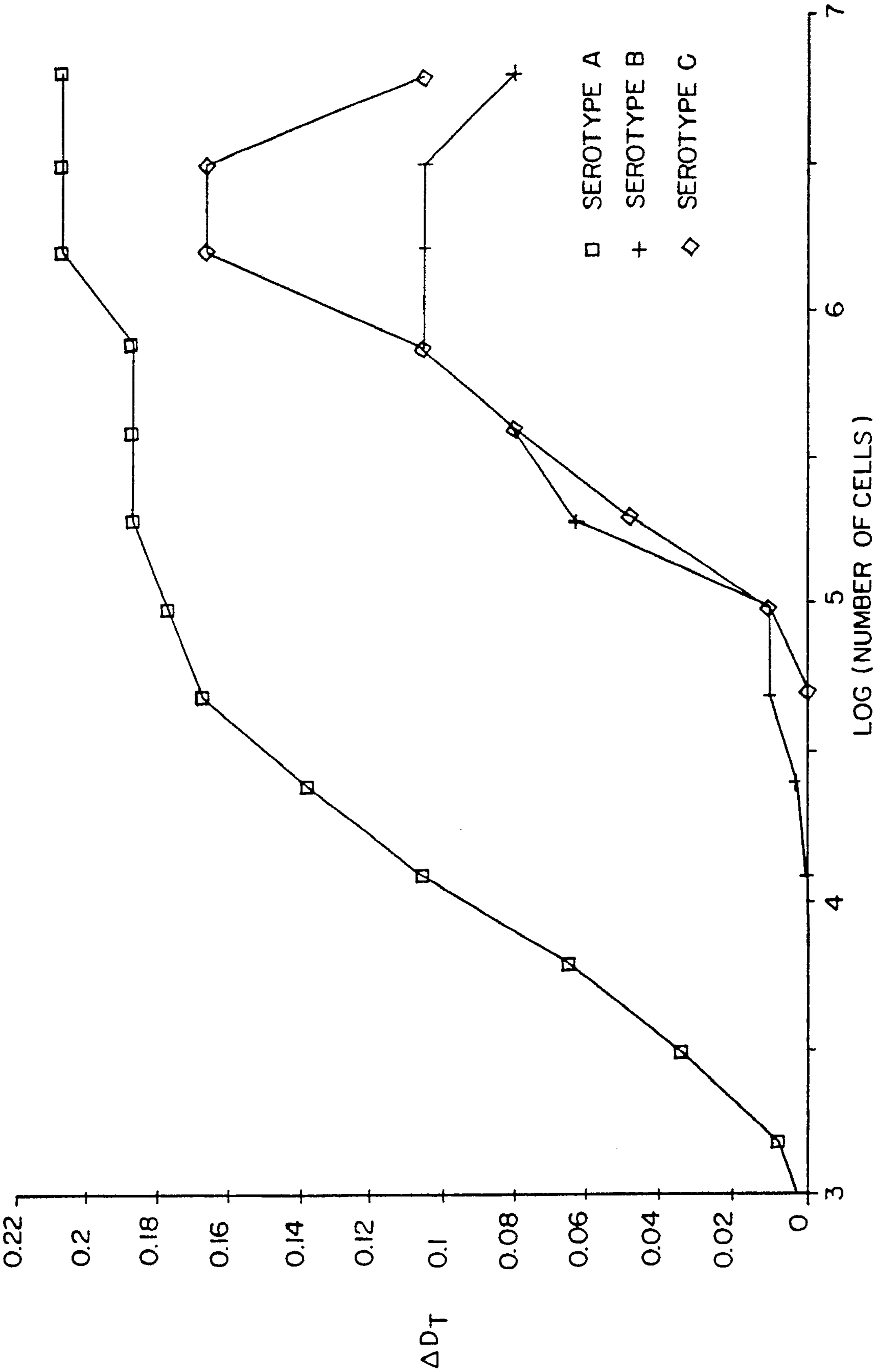

ΔD_T
0.22
0.2
0.18
0.16
0.14
0.12
0.1
0.08
0.06
0.04
0.02
0
3
4
5
6
7
LOG (NUMBER OF CELLS)
SEROTYPE A
SEROTYPE B
SEROTYPE C


FIG.8

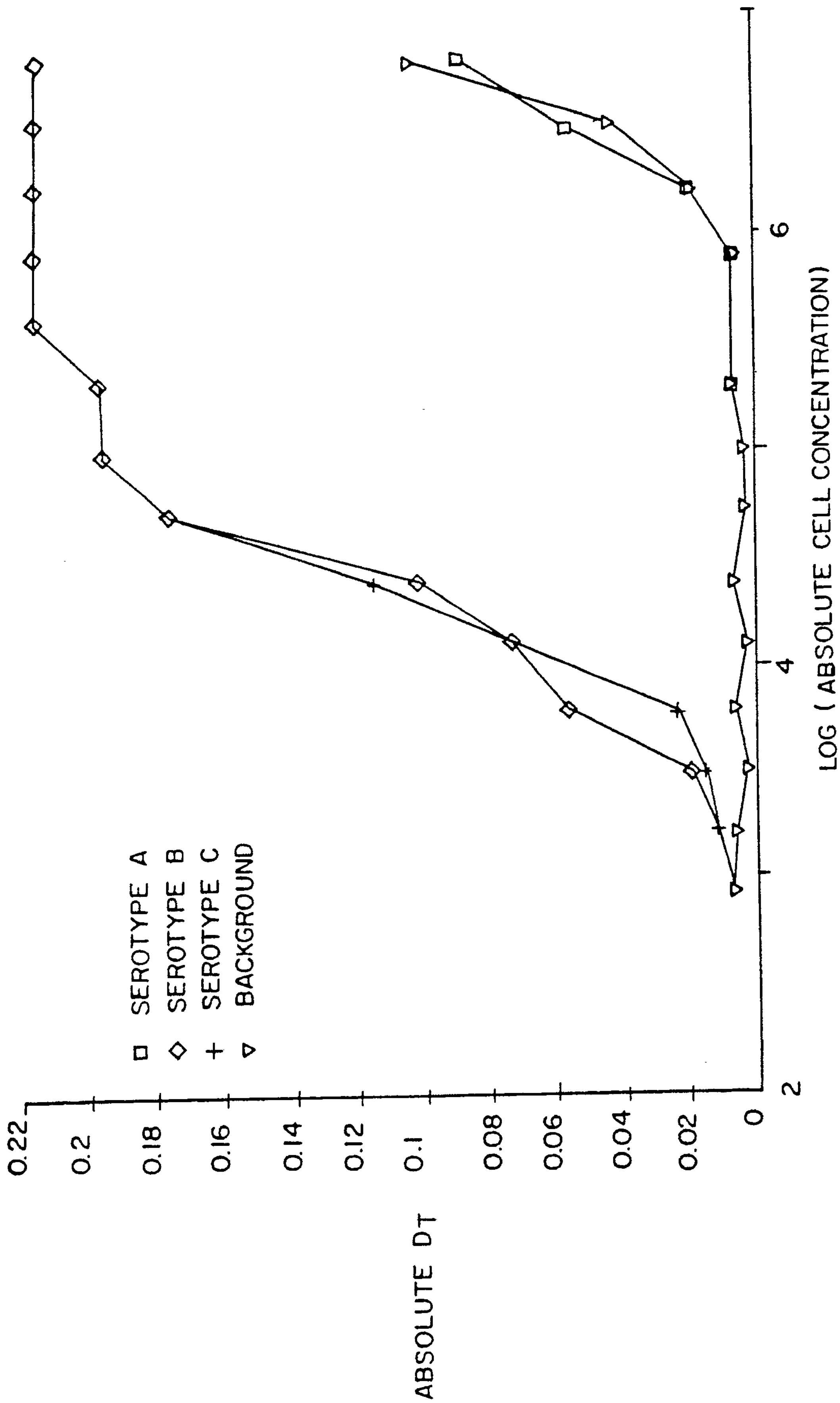
SEROTYPE A
SEROTYPE B
SEROTYPE C
BACKGROUND
ABSOLUTE DT
0.22
0.2
0.18
0.16
0.14
0.12
0.1
0.08
0.06
0.04
0.02
0
2
4
6
LOG ( ABSOLUTE CELL CONCENTRATION)

FIG.9

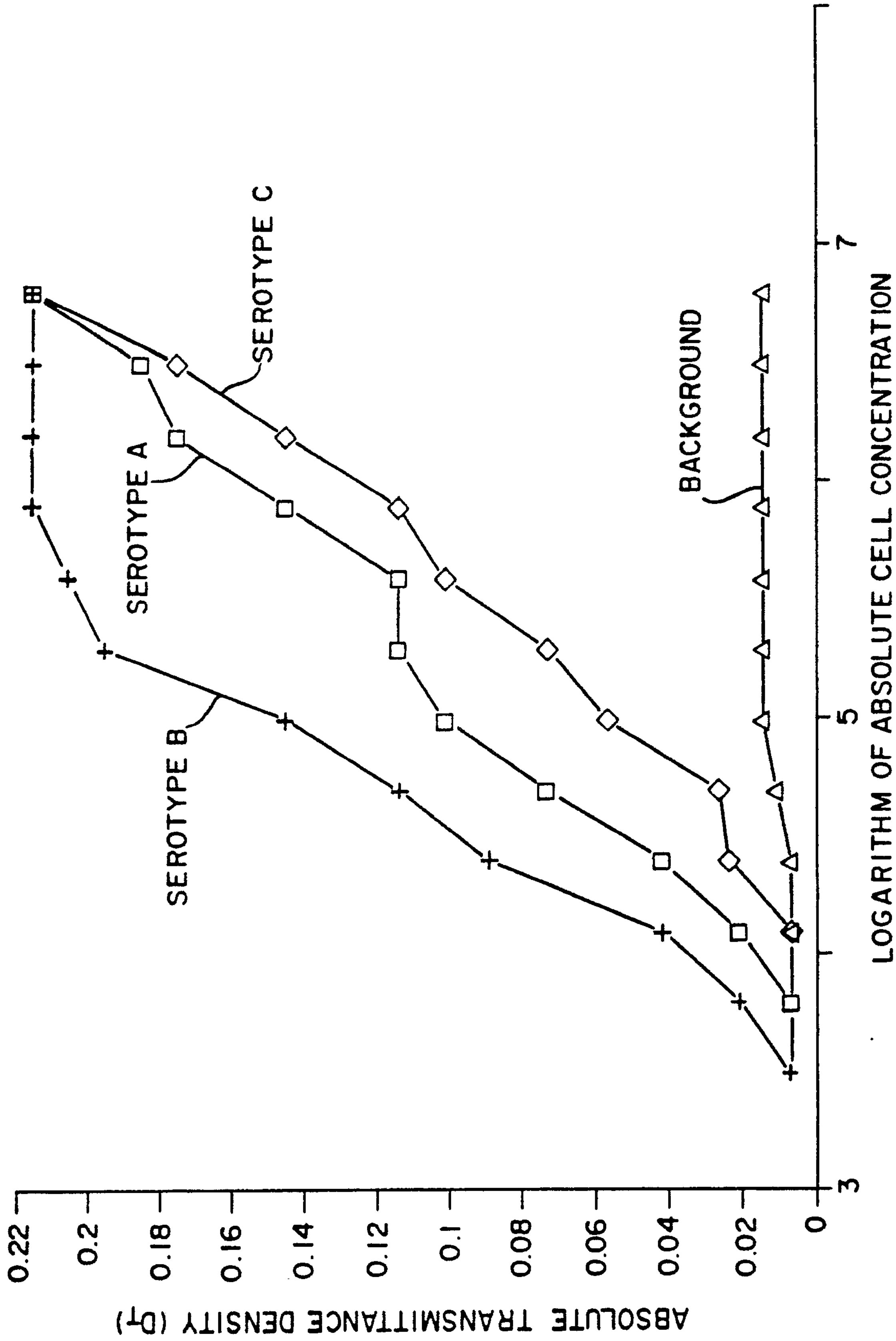
SEROTYPE C
SEROTYPE A
SEROTYPE B
BACKGROUND
ABSOLUTE TRANSMITTANCE DENSITY (DT)
0.22
0.2
0.18
0.16
0.14
0.12
0.1
0.08
0.06
0.04
0.02
0
3
5
7
LOGARITHM OF ABSOLUTE CELL CONCENTRATION

FIG. 10

European Patent Office

# EUROPEAN SEARCH REPORT

Application Number

**EP 91 20 0047**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 269 388 (MINNESOTA MINING AND MANUFACTURING CO.)<br>* Page 3, lines 63-64; page 13, examples 6,7; page 15, example 9 * | 1-12 | G 01 N 33/569<br>G 01 N 33/543<br>G 01 N 33/546 |
| Y | EP-A-0 253 464 (HYBRITECH INC.)<br>* Page 8, lines 22-29; figure 3 * | 1-12 | |
| T | JOURNAL OF DENTAL RESEARCH (US), vol. 69, special issue March 1990, page 243, abstract no. 1077, Eastman Kodak, Co., Rochester, NY, US; P. CONTESTABLE et al.: "A rapid enzyme immunoassay for three suspected periodontal pathogens"<br>* Whole abstract * | 1-12 | |
| A | EP-A-0 335 244 (ABBOTT LABORATORIES)<br>* Page 5, line 1 - page 6, line 40; page 8, lines 20-37 * | 7,12 | |

TECHNICAL FIELDS SEARCHED (Int. Cl.5)

G 01 N

The present search report has been drawn up for all claims

| Place of search | Date of completion of search | Examiner |
|---|---|---|
| The Hague | 19 April 91 | VAN BOHEMEN C.G. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document